(19) **Europäisches Patentamt / European Patent Office / Office européen des brevets**

(11) **EP 3 837 526 B1**

(12) **FASCICULE DE BREVET EUROPEEN**

(45) Date de publication et mention de la délivrance du brevet:
**09.07.2025  Bulletin 2025/28**

(21) Numéro de dépôt: **19759016.9**

(22) Date de dépôt: **05.08.2019**

(51) Classification Internationale des Brevets (IPC):
**G01N 15/14** (2024.01)     **G01N 21/3563** (2014.01)
**G01N 21/39** (2006.01)     **G01N 21/65** (2006.01)
**G01N 15/10** (2024.01)

(52) Classification Coopérative des Brevets (CPC):
**G01N 15/1468; G01N 15/1434; G01N 21/3563;**
G01N 21/39; G01N 2015/1006; G01N 2015/1472

(86) Numéro de dépôt international:
**PCT/FR2019/051904**

(87) Numéro de publication internationale:
**WO 2020/035646 (20.02.2020 Gazette 2020/08)**

(54) **PROCEDE D'OBSERVATION D'UN ECHANTILLON DANS LE DOMAIN INFRAROUGE**

VERFAHREN ZUR BEOBACHTUNG EINER PROBE IN DER INFRAROTSPEKTRUM

METHOD FOR OBSERVING A SAMPLE IN THE INFRARED SPECTRUM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **14.08.2018  FR 1857483**

(43) Date de publication de la demande:
**23.06.2021  Bulletin 2021/25**

(73) Titulaire: **Commissariat à l'Energie Atomique et aux Energies Alternatives**
**75015 Paris (FR)**

(72) Inventeurs:
• **COUTARD, Jean-Guillaume**
  **38054 Grenoble Cedex 9 (FR)**
• **ALLIER, Cédric**
  **38054 Grenoble Cedex 9 (FR)**
• **BECKER, Sébastien**
  **38054 Grenoble Cedex 9 (FR)**
• **DUPOY, Mathieu**
  **38054 Grenoble Cedex 9 (FR)**

(74) Mandataire: **INNOV-GROUP**
**209 Avenue Berthelot**
**69007 Lyon (FR)**

(56) Documents cités:
**FR-A1- 3 034 197     US-A1- 2012 122 084**

• **OZCAN AYDOGAN ET AL: "Lensless Imaging and Sensing", ANNUAL REVIEW OF BIOMEDICAL ENGINEERING, vol. 18, no. 1, 11 July 2016 (2016-07-11), US, pages 77 - 102, XP055789975, ISSN: 1523-9829, Retrieved from the Internet <URL:https://www.annualreviews. org/doi/pdf/10.1146/ annurev-bioeng-092515-010849> [retrieved on 20250124], DOI: 10.1146/annurev-bioeng-092515-010849**
• **LANGE D ET AL: "A microfluidic shadow imaging system for the study of the nematode Caenorhabditis elegans in space", SENSORS AND ACTUATORS B: CHEMICAL, ELSEVIER BV, NL, vol. 107, no. 2, 29 June 2005 (2005-06-29), pages 904 - 914, XP027810555, ISSN: 0925-4005, [retrieved on 20050629]**
• **RICHARDS P L: "BOLOMETERS FOR INFRARED AND MILLIMETER WAVES", JOURNAL OF APPLIED PHYSICS, AMERICAN INSTITUTE OF PHYSICS, US, vol. 76, no. 1, 1 July 1994 (1994-07-01), pages 1 - 24, XP000459427, ISSN: 0021-8979, DOI: 10.1063/1.357128**

EP 3 837 526 B1

## Description

## DOMAINE TECHNIQUE

Le domaine technique de l'invention est lié à l'observation d'un échantillon, en particulier un échantillon biologique, l'observation étant réalisée à des fins d'analyse histologique. L'observation de l'échantillon est réalisée selon une configuration d'imagerie sans lentille.

## ART ANTERIEUR

La caractérisation d'échantillons biologiques par imagerie spectrale infrarouge est à présent une technologie largement décrite dans la littérature pour ses applications dans le diagnostic, et en particulier dans le domaine de l'histopathologie. Elle permet d'obtenir, sans marquage, des informations biomoléculaires relatives à des cellules ou à des tissus. Cette méthode est basée sur une signature spectrale d'un échantillon vis-à-vis d'une pathologie. Lorsqu'un faisceau lumineux traversant l'échantillon présente une longueur d'onde correspondant à une énergie entre deux niveaux de vibration moléculaire, une partie du faisceau est absorbée. Ainsi, par spectrométrie d'absorption, on peut estimer une absorbance spectrale de l'échantillon, permettant l'obtention d'une information sur la composition moléculaire de ce dernier. Le spectre de l'absorbance constitue en cela une signature moléculaire de l'échantillon.

Cependant, dans ce type de méthode, il est nécessaire de balayer l'échantillon avec un faisceau laser. La taille du faisceau laser conditionne en effet la résolution spatiale de la mesure. Aussi, lorsqu'on souhaite obtenir une information spectrale spatialement résolue, le faisceau laser doit être fin. Il en résulte qu'une analyse d'une surface d'échantillon de quelques $mm^2$ ou quelques $cm^2$ est longue. De plus, l'instrumentation liée à l'imagerie spectrale infrarouge est complexe et onéreuse.

Le document US2012/0122084A1 décrit un dispositif de caractérisation de cellules, en mesurant une absorption, dans le domaine de l'infra-rouge, à l'aide d'un photodétecteur infra-rouge. Un capteur d'image visible permet de localiser la cellule, ou de déterminer son orientation.

En dehors du domaine de l'infrarouge, l'observation d'échantillons, et en particulier des échantillons biologiques, par imagerie sans lentille, connaît un développement important depuis ces dix dernières années. Cette technique permet d'observer un échantillon en le disposant entre une source de lumière et un capteur d'image, sans disposer de lentille de formation d'image entre l'échantillon et le capteur d'image. Ainsi, le capteur d'image collecte une image d'une onde lumineuse transmise par l'échantillon, sans conjugaison entre le capteur d'image et l'échantillon.

Le document WO2008090330 décrit par exemple un dispositif permettant l'observation de particules biologiques, par imagerie sans lentille. Les particules biologiques sont par exemple des cellules. Le dispositif permet d'associer, à chaque cellule, une figure d'interférence dont la morphologie permet d'identifier le type de cellule. L'imagerie sans lentille apparaît alors comme une alternative simple, et peu onéreuse, à un microscope classique. De plus, elle confère un champ d'observation nettement plus important que ne peut l'être celui d'un microscope. Dans le domaine visible, l'imagerie sans lentille a été appliquée pour examiner des lames de tissus, de type lames d'anatomo-pathologie. Des exemples d'application ont par exemple été décrits dans WO2016189257 ou dans EP3199941. On accède alors à des images exploitables de l'échantillon, mais ces images ne comportent pas d'information moléculaire, ou d'information relative au type de cellules présentes dans l'échantillon.

Dans le domaine de l'infrarouge, une application de l'imagerie sans lentille est présentée dans EP3147646. Dans ce document, on décrit un procédé permettant de former une image d'une particule. L'image obtenue permet d'observer une particule.

Une autre approche de l'imagerie sans lentille est décrite dans US20050190286. Dans ce document, on décrit une formation d'une image de particules déposées au contact de pixels d'un capteur d'image. Cette méthode est désignée par le terme ombroscopie. L'image formée par le capteur d'image comporte des ombres de chaque particule, ce qui permet d'accéder à la forme de chaque particule. On peut alors identifier les particules en fonction de leur forme.

D'autres publications pertinentes comprennent: FR3034197A1, LANGE D ET AL: "A microfluidic shadow imaging system for the study of the nematode Caenorhabditis elegans in space",SENSORS AND ACTUATORS B: CHEMICAL, ELSEVIER BV, NL, vol. 107, no. 2, 29 juin 2005 (2005-06-29), pages 904-914, ISSN: 0925-4005;

RICHARDS P L: "BOLOMETERS FOR INFRARED AND MILLIMETER WAVES",JOURNAL OF APPLIED PHYSICS, AMERICAN INSTITUTE OF PHYSICS, US, vol. 76, no. 1, 1 juillet 1994 (1994-07-01), pages 1-24, ISSN: 0021-8979, DOI: 10.1063/1.357128; et Ozcan Aydogan ET AL: "Lensless Imaging and Sensing",Annual Review of Biomedical Engineering, vol. 18, no. 1, 11 juillet 2016 (2016-07-11), pages 77-102, US ISSN: 1523-9829, DOI: 10.1146/annurev-bioeng-092515-010849Extrait de l'Internet:URL:https://www.annualreviews.org/doi/pdf/10.1146/annurev-bioeng-092515-010849

Les inventeurs proposent une méthode permettant une caractérisation d'un échantillon, en particulier une lame de tissu, en utilisant un dispositif relativement simple. La méthode permet d'adresser un champ d'observation important, ce qui permet d'obtenir un résultat rapide. Elle permet d'obtenir une cartographie de l'échantillon, pour établir des informations relatives à des molécules ou à des liaisons moléculaires. La cartographie réalisée peut être utilisée en vue d'établir un diagnostic.

## EXPOSE DE L'INVENTION

Un objet de l'invention est un procédé d'observation d'un échantillon, notamment un échantillon biologique, l'échantillon étant disposé entre une source de lumière et un capteur d'image pixelisé, la source de lumière émettant un faisceau lumineux incident, se propageant jusqu'à l'échantillon selon un axe de propagation, et selon une longueur d'onde d'émission comprise entre 1 $\mu$m et 20 $\mu$m, le procédé comportant les étapes suivantes :

    a) illumination de l'échantillon par la source de lumière ;
    b) acquisition d'une image de l'échantillon par le capteur d'image pixelisé, aucune optique de formation d'image n'étant disposée entre l'échantillon et le capteur d'image ;

le procédé étant caractérisé en ce que l'échantillon est apte à absorber une partie du faisceau lumineux incident, de telle sorte que l'image acquise est représentative d'une absorption du faisceau incident par l'échantillon, à la longueur d'onde d'émission.

De préférence, la longueur d'onde d'émission est comprise entre 5 $\mu$m et 20 $\mu$m. De préférence, la source de lumière est une source de lumière laser.

Selon un mode de réalisation, le procédé comporte également les étapes suivantes :

    c) illumination du capteur d'image par la source de lumière, à la longueur d'onde d'émission, sans échantillon entre le capteur d'image et la source de lumière, de façon à obtenir une image de fond ;
    d) comparaison de l'image acquise lors de l'étape b) et de l'image de fond acquise lors de l'étape c) pour obtenir une image de l'absorbance de l'échantillon à la longueur d'onde d'émission.

La comparaison peut notamment prendre la forme d'un ratio.

Selon un mode de réalisation, la longueur d'onde d'émission est une longueur d'onde d'absorption d'un analyte, correspondant à un pic d'absorption de l'analyte, le procédé comportant une cartographie d'une quantité de l'analyte dans l'échantillon à partir de l'image de l'absorbance à la longueur d'onde d'émission. Par pic d'absorption d'un analyte, on entend une plage de longueurs d'onde correspondant à un maximum local d'absorption.

Selon un mode de réalisation, la longueur d'onde d'émission est une longueur d'onde d'absorption d'un analyte, correspondant à un pic d'absorption de l'analyte, de façon à obtenir une image de l'absorbance de l'échantillon à la longueur d'onde d'absorption, le procédé comportant les étapes suivantes :

    e) illumination de l'échantillon selon une longueur d'onde de base, à laquelle l'absorption de l'analyte est inférieure à l'absorption de l'analyte à la longueur d'onde d'absorption;
    f) acquisition d'une image de l'échantillon par le capteur d'image pixelisé ;
    g) illumination du capteur d'image par la source de lumière, à la longueur d'onde de base, sans échantillon entre le capteur d'image et la source de lumière, de façon à obtenir une image de fond à la longueur d'onde de base ;
    h) comparaison de l'image acquise lors de l'étape f) et de l'image de fond acquise lors de l'étape g) pour obtenir une image de l'absorbance de l'échantillon à la longueur d'onde de base.

Par absorption de l'analyte, on entend une absorption du faisceau lumineux incident par l'analyte.

La longueur d'onde de base est de préférence une longueur d'onde voisine du pic d'absorption de l'analyte. Il peut notamment s'agir d'une longueur d'onde définissant une ligne de base du pic d'absorption.

Le procédé peut alors comporter une soustraction des images d'absorbance de l'échantillon respectivement à la longueur d'onde d'absorption et à la longueur d'onde de base, de manière à obtenir une image d'absorbance due à l'analyte.

Selon un mode de réalisation, les étapes a) à d) sont répétées en illuminant successivement l'échantillon selon :

-     une première longueur d'onde d'absorption, correspondant à une longueur d'onde d'absorption d'un premier analyte ;
-     une deuxième longueur d'onde d'absorption, correspondant à une longueur d'onde d'absorption d'un deuxième analyte ;

de manière à obtenir des images de l'absorbance de l'échantillon respectivement à la première longueur d'onde d'absorption et à la deuxième longueur d'onde d'absorption.

Les étapes e) à h) peuvent également être répétées en illuminant successivement l'échantillon selon :

-     une première longueur d'onde de base, à laquelle l'absorption du premier analyte est inférieure à l'absorption du premier analyte à la première longueur d'onde d'absorption ($\lambda_{a,1}$);
-     une deuxième longueur d'onde de base, à laquelle l'absorption du deuxième analyte est inférieure à l'absorption du deuxième analyte à la deuxième longueur d'onde d'absorption;

de manière à obtenir des images de l'absorbance de l'échantillon respectivement à la première longueur d'onde de base et à la deuxième longueur d'onde de base.

Le procédé peut également comporter :

- une soustraction des images d'absorbance de l'échantillon respectivement à la première longueur d'onde d'absorption et à la première longueur d'onde de base, de manière à obtenir une image d'absorbance due au premier analyte ;
- une soustraction des images d'absorbance de l'échantillon respectivement à la deuxième longueur d'onde d'absorption et à la deuxième longueur d'onde de base, de manière à obtenir une image d'absorbance due au deuxième analyte ;
- une comparaison de l'image d'absorbance due au premier analyte et de l'image d'absorbance due au deuxième analyte.

Le procédé peut comporter l'une des caractéristiques suivantes, prises isolément ou selon les combinaisons techniquement réalisables :

- l'échantillon est une lame de tissu biologique ;
- la source de lumière éclaire une surface de l'échantillon supérieure à 1 mm² ou supérieure à 5 mm² ;
- l'image de l'échantillon acquise par le capteur d'image correspond à une surface d'échantillon supérieure à 1 mm² ou supérieure à 5 mm² ;
- les pixels du capteur d'image définissent un plan de détection, l'échantillon étant disposé à une distance du plan de détection inférieure à 1 mm ;
- le procédé comporte, à partir de l'image acquise, une étape de détermination d'au moins une région d'intérêt de l'échantillon.

L'échantillon peut être maintenu par un support comportant au moins un des matériaux suivants:

- silicium ;
- et/ou germanium ;
- et/ou fluorure de calcium ;
- et/ou fluorure de baryum.

Un autre objet de l'invention est un dispositif d'observation d'un échantillon, comportant une source de lumière émettant dans une bande spectrale comprise entre 1 $\mu$m et 20 $\mu$m, un capteur d'image sensible dans ladite bande spectrale, et un élément de maintien de l'échantillon, apte à recevoir un échantillon, l'élément de maintien d'échantillon étant configuré de telle sorte que l'échantillon, lorsqu'il est disposé sur l'élément de maintien, s'étend entre une source de lumière et un capteur d'image, le dispositif étant tel qu'aucune optique de formation d'image n'est disposée entre l'échantillon, lorsqu'il est disposé sur l'élément de maintien d'échantillon, et le capteur d'image. Le dispositif peut comporter une unité de traitement, par exemple un microprocesseur, configurée pour recevoir au moins une image acquise par le capteur d'image, et pour mettre en œuvre les opérations de traitement d'images décrites ci-dessus ou ci-après. D'autres avantages et caractéristiques ressortiront plus clairement de la description qui va suivre de modes particuliers de réalisation de l'invention, donnés à titre d'exemples non limitatifs, et représentés sur les figures listées ci-dessous.

## FIGURES

La figure 1 représente un dispositif permettant une mise en œuvre de l'invention.

La figure 2A est une image visible d'un premier échantillon. La figure 2B montre des images du premier échantillon obtenues en mettant en œuvre l'invention. La figure 2B est représentative d'une absorption du premier échantillon à une longueur d'onde de 7.35 $\mu$m.

La figure 3A schématise un pic d'absorption d'un analyte. La figure 3B comporte les principales étapes d'un mode de réalisation de l'invention. La figure 3C est une image de l'absorbance d'un deuxième échantillon à un nombre d'onde de 1081 cm$^{-1}$ (soit $\lambda$ = 9.2 $\mu$m). La figure 3D est une image de l'absorbance du deuxième échantillon à un nombre d'onde de 1654 cm$^{-1}$ (soit À = 6 $\mu$m).

## EXPOSE DE MODES DE REALISATION PARTICULIERS

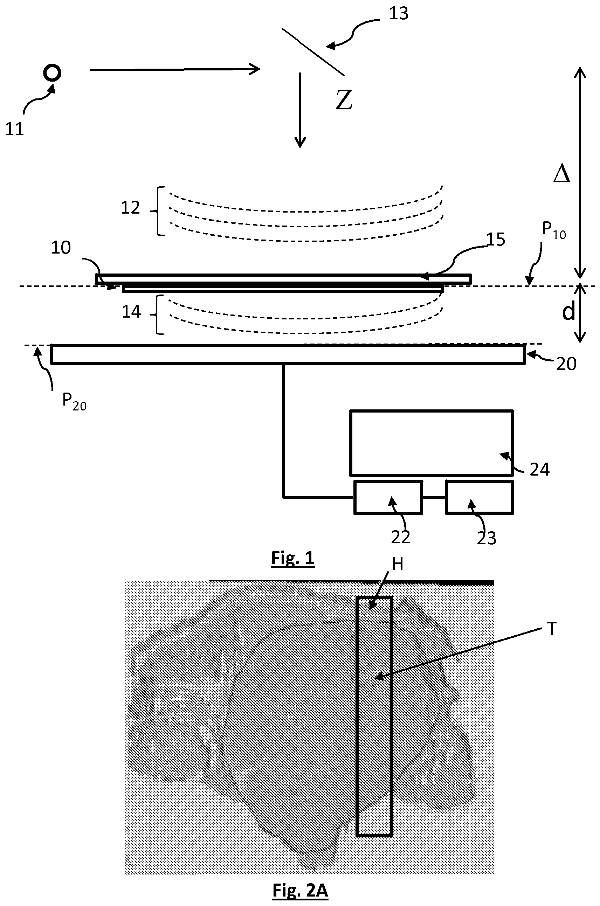

La figure 1 représente un exemple de dispositif permettant une mise en œuvre de l'invention. Une source de lumière 11 est configurée pour émettre un faisceau lumineux 12, dit faisceau lumineux incident, se propageant en direction d'un échantillon 10. Le faisceau lumineux incident atteint l'échantillon en se propageant selon un axe de propagation Z.

Dans cet exemple, l'échantillon 10 un échantillon biologique que l'on souhaite caractériser. Il peut notamment s'agir d'une lame de tissu destinée à une analyse histologique, ou lame d'anatomo-pathologie, comportant une fine épaisseur de tissu déposée sur une lame transparente 15 faisant office de support d'échantillon. Par fine épaisseur, on entend une épaisseur de préférence inférieure à 100 $\mu$m, et de préférence inférieure à 10 $\mu$m, typiquement quelques micromètres. L'échantillon s'étend selon un plan P$_{10}$, dit plan de l'échantillon. Le plan de l'échantillon est de préférence perpendiculaire ou sensiblement perpendiculaire à l'axe de propagation Z. Le terme sensiblement perpendiculaire signifie perpendiculaire en admettant une tolérance angulaire de quelques degrés, inférieure à 20° ou 10°.

La lame de tissu 10 est obtenue selon des procédés de préparation connus, à partir d'un prélèvement tissulaire extrait par biopsie ou frottis. Le prélèvement est ensuite préparé de façon à se présenter sous la forme d'une fine épaisseur déposée sur la lame transparente 15. De tels procédés sont connus dans le domaine de l'histologie. Ils comportent par exemple une découpe d'un tissu congelé, ou une inclusion d'un tissu prélevé dans une matrice de paraffine. De préférence, l'échantillon n'a subi aucun marquage préalable, à l'aide d'un marqueur exogène ajouté à l'échantillon avant son analyse.

L'échantillon peut comporter un analyte, dont on souhaite évaluer une distribution spatiale dans l'échantillon. Par analyte, on entend par exemple une molécule ou une partie d'une molécule ou une liaison moléculaire.

La lame 15 est transparente vis-à-vis du faisceau incident 12. Elle peut comprendre ou être constituée de matériaux tels le silicium, le germanium, le fluorure de calcium ($CaF_2$), le fluorure de baryum ($BaF_2$).

La distance $\Delta$ entre la source de lumière et l'échantillon, selon l'axe Z, est de préférence supérieure à 1 cm. Elle est de préférence comprise entre 2 et 30 cm. De préférence, la source de lumière, vue par l'échantillon, est considérée comme ponctuelle. Cela signifie que son diamètre (ou sa diagonale) est préférentiellement inférieur au dixième, mieux au centième de la distance entre l'échantillon et la source de lumière. Ainsi, de préférence, la lumière parvient à l'échantillon sous la forme d'ondes planes, ou pouvant être considérées comme telles.

La source de lumière 11 est une source émettant dans l'infrarouge. Il peut s'agir de l'infrarouge court, usuellement désigné par l'acronyme SWIR (Short Wavelenght Infrared), s'étendant entre 1 et 3 $\mu$m, ou du moyen infrarouge, usuellement désigné par l'acronyme MWIR (Medium Wavelenght Infrared), s'étendant entre 3 et 5 $\mu$m, ou encore de l'infrarouge long, usuellement désigné par l'acronyme LWIR (Long Wavelenght Infrared), s'étendant entre 8 et 20 $\mu$m. Ainsi, d'une façon générale, le faisceau incident 12 est émis selon une longueur d'onde $\lambda$ s'étendant entre 1 $\mu$m et 20 $\mu$m, ce qui correspond à un nombre d'onde (1/$\lambda$) compris entre 500 cm$^{-1}$ et 10000 cm$^{-1}$. Dans l'exemple représenté, le dispositif comporte un réflecteur 13, pour réfléchir le faisceau lumineux 12 émis par la source de lumière 11 vers l'échantillon. De préférence, la longueur d'onde est comprise entre 5$\mu$m et 20 $\mu$m.

La source de lumière 11 est de préférence une source laser. Il peut notamment s'agir d'une source laser accordable en longueur d'onde, par exemple un laser QCL, acronyme de Quantum Cascade Laser, signifiant laser à cascade quantique, en particulier un laser à cavité externe. La largeur de la bande spectrale d'émission de la source de lumière est de préférence inférieure à 50 nm, voire à 10 nm, voire à 5 nm. Une source de lumière peut comporter plusieurs sources laser élémentaires QCL, émettant respectivement dans différentes bandes spectrales.

L'échantillon 10 est disposé entre la source de lumière 11 et un capteur d'image 20. Ce dernier s'étend de préférence parallèlement, ou sensiblement parallèlement à la lame transparente 15 supportant l'échantillon. Le terme sensiblement parallèlement signifie que les deux éléments peuvent ne pas être rigoureusement parallèles, une tolérance angulaire de quelques degrés, inférieure à 20° ou 10° étant admise. L'échantillon est disposé sur un élément de maintien, configuré pour maintenir l'échantillon, et son support, entre la source de lumière et le capteur d'image.

Sous l'effet de l'illumination par le faisceau lumineux 12, se propageant selon l'axe de propagation Z jusqu'à l'échantillon, ce dernier transmet une onde lumineuse 14, dite onde lumineuse transmise. L'onde lumineuse transmise 14 se propage, parallèlement à l'axe Z, jusqu'à un capteur d'image 20. L'échantillon absorbe une partie du faisceau lumineux 12. Aussi, l'onde lumineuse transmise 14 correspond à une partie du faisceau lumineux 12 non absorbée par l'échantillon.

Le capteur d'image 20 est apte à former une image de l'onde lumineuse transmise 14 selon un plan de détection $P_{20}$. Dans cet exemple, le capteur d'image est formé par une matrice de bolomètres, chaque bolomètre de la matrice présentant une bande spectrale de détection comprise entre 5 $\mu$m et 20 $\mu$m. Chaque bolomètre forme un pixel. Dans les exemples décrits par la suite, chaque pixel est formé par un bolomètre encapsulé sous vide. De façon classique, on peut acquérir une image de noir, correspondant au bruit de chaque bolomètre en l'absence d'illumination. L'image de noir est ensuite soustraite de chaque image acquise. Lorsque l'échantillon est disposé entre le capteur d'image et la source de lumière, l'image acquise par le capteur d'image est représentative de l'absorption du faisceau incident 12 par l'échantillon 10.

Lorsque le capteur d'image 20 comporte des pixels non fonctionnels, ou pixels morts, l'intensité de chaque pixel non fonctionnel est remplacée par une moyenne des intensités mesurées par les pixels adjacents au pixel non fonctionnel.

La distance d entre chaque pixel et l'échantillon 10 est de préférence inférieure à 5 mm. Plus elle est réduite, meilleure est la résolution spatiale de l'image acquise par le capteur d'image. Aussi, il est avantageux que la distance d soit inférieure à 1 mm, voire inférieure à 500 $\mu$m.

L'échantillon peut être déposé au contact direct des pixels du capteur d'image 20, selon une configuration d'ombroscopie, telle que décrite dans US20050190286.

Du fait de l'absence d'optique de formation d'image entre l'échantillon 10 et le capteur d'image 20, le champ d'observation du capteur d'image est défini par la taille du capteur d'image et la taille du faisceau incident. Le champ d'observation peut être supérieur à 1 mm$^2$, voire supérieur à 5 mm$^2$ ou 10 mm$^2$. De ce fait, l'acquisition d'une seule image permet d'obtenir simultanément une intensité de l'onde lumineuse 14 transmise par plusieurs mm² de l'échantillon, typiquement au moins 5 ou 10 mm² de l'échantillon.

De préférence, la lame transparente 15 comporte un revêtement antireflet. Par exemple, lorsque la lame transparente est constituée de silicium, elle peut comporter une couche mince de germanium ou de sulfure de zinc (ZnS). Cela permet de limiter l'apparition de franges d'interférence sur les images formées par le capteur d'image. En l'absence d'une couche mince antireflet, la lame transparente 15 se comporter comme une cavité de type Fabry-Perot, ce qui conduit à la formation de franges d'interférence indésirables sur l'image acquise par le capteur d'image 20.

Une unité de traitement, prenant par exemple la forme d'un microprocesseur 22, est configurée pour effectuer, à partir des images acquises par le capteur d'image 20, des opérations de traitement d'images telles que décrites par la suite. Le microprocesseur 22 est relié à une mémoire 23, comportant des instructions relatives aux traitements d'images à effectuer. Il peut être relié à un écran 24.

La figure 2A représente un premier échantillon ayant été examiné à l'aide d'un dispositif tel que décrit sur la figure 1. Il s'agit d'une coupe de tissu musculaire prélevé d'une souris, puis congelé. Le tissu prélevé comportait une tumeur suite à l'injection de cellules tumorales de type CAL33. Il a été découpé pour obtenir un échantillon d'épaisseur égale à 4 $\mu$m. L'échantillon a ensuite été déposé sur une lame de silicium ou sur une lame de CaF$_2$ (Fluorure de Calcium).

Les paramètres expérimentaux étaient :

- source de lumière : source laser QCL émettant à la longueur d'onde de 7.35 $\mu$m.
- diamètre du faisceau incident : 1.5 mm ;
- capteur d'image : matrice de 80x80 bolomètres de 17 $\mu$m de côté, avec un écart centre-à-centre de chaque pixel égal à 30 $\mu$m, conférant un champ d'observation d'environ 2.4 x 2.4 mm².

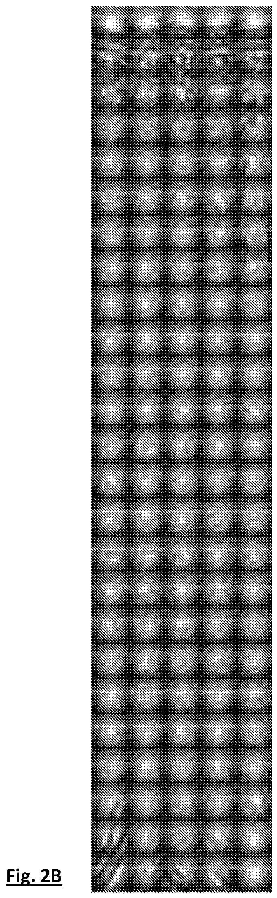

La lame 15 a été montée sur une platine de translation, de façon à être translatée selon deux directions orthogonales X et Y perpendiculairement à l'axe de propagation Z. Le balayage a permis de constituer une matrice de 150 images agencées selon 25 lignes et 6 colonnes. Le champ couvert par la matrice d'images correspond à une surface d'échantillon de largeur 3 mm et de longueur 12.5 mm. La surface de l'échantillon observée est encadrée sur la figure 2A. La zone centrale de l'échantillon, repérée par la lettre T, correspond à une tumeur maligne tandis que la zone périphérique, repérée par la lettre H, correspond à un tissu sain. La figure 2B montre les images acquises par le capteur d'image.

On observe que les images correspondant à la zone tumorale T ont un aspect dense, tandis que les images correspondant à la zone saine H ont un aspect spongieux. Ainsi, une image infrarouge, acquise selon une modalité d'imagerie sans lentille, permet de détecter la présence d'une zone tumorale et de la visualiser. Elle permet de définir, sur l'échantillon, une région d'intérêt correspondant à la zone tumorale ou à la zone saine.

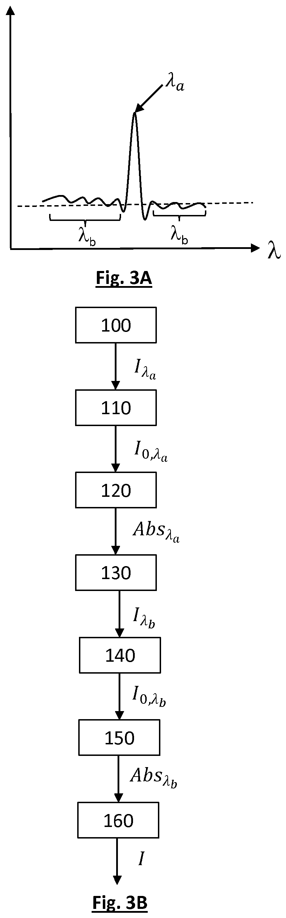

Les figures 3A à 3D correspondent à un deuxième mode de réalisation, dans lequel plusieurs images d'un échantillon sont réalisées, en modulant la longueur d'onde $\lambda$ du faisceau d'illumination 12.

Il est connu que la transmittance spectrale de la lumière d'un échantillon varie en fonction de la composition de ce dernier, en raison de la présence de pics d'absorption correspondant à des modes de vibrations de molécules composant l'échantillon. La présence de pics d'absorption est la base des méthodes de spectrométrie vibrationnelle de type spectroscopie infrarouge ou spectrométrie Raman.

Par transmittance $tr_{\lambda_i}$, on entend un ratio entre une intensité $i_{\lambda_i}$ de l'onde lumineuse 14 transmise par l'échantillon, et détectée par le capteur d'image, à la longueur d'onde $\lambda_i$, sur une intensité de l'onde lumineuse détectée par le capteur d'image, à la même longueur d'onde, en l'absence d'échantillon.

Ainsi, selon la loi de Beer-Lambert :

$$tr_{\lambda_i} = \frac{i_{\lambda_i}}{i_{0,\lambda_i}}(1)$$

où $i_{0,\lambda_i}$ est l'intensité détectée par le capteur d'image en l'absence d'échantillon.

L'absorbance $abs_{\lambda_i}$ à la longueur d'onde $\lambda_i$ est obtenue selon l'expression :

$$abs_{\lambda_i} = -ln\left(tr_{\lambda_i}\right) = -ln\left(\frac{i_{\lambda_i}}{i_{0,\lambda_i}}\right)(1')$$

La figure 3A montre un exemple de spectre d'absorption d'un analyte, l'axe des abscisses représentant la longueur d'onde. On distingue un pic d'absorption, repéré par une flèche, à une longueur d'onde d'absorption $\lambda_a$. De part et d'autre du pic d'absorption s'étend une ligne de base, repérée par une droite en pointillés. La ligne de base correspond à des longueurs d'onde de base $\lambda_b$, s'étendant de part et d'autre du pic d'absorption. Ainsi, une longueur d'onde de base est une longueur d'onde située en dehors du pic d'absorption de l'analyte. De préférence, une longueur d'onde de base délimite le pic d'absorption. A la longueur d'onde de base, l'absorption du faisceau lumineux incident par l'analyte est inférieure à l'absorption du faisceau incident par l'analyte dans le pic d'absorption.

La démarche proposée par les inventeurs consiste à déterminer une cartographie d'une absorbance résultant de la présence d'un analyte, dont la longueur d'onde d'absorption $\lambda_a$ est connue. Pour ce faire, le procédé consiste à :

- acquérir une image, représentative de l'absorption du faisceau lumineux 12 par l'échantillon, correspondant à une image $I_{\lambda_a}$ acquise par le capteur d'image lorsque le faisceau d'illumination est émis selon la longueur d'onde d'absorption de l'analyte $\lambda_a$. Une telle image est désignée par le terme image d'absorption.

- acquérir une image, dite image de fond, correspondant à une image $I_{0,\lambda_a}$ acquise par le capteur d'image en l'absence d'échantillon, à la longueur d'onde d'absorption $\lambda_a$.

La comparaison de l'image d'absorption $I_{\lambda_a}$ et de l'image de fond $I_{0,\lambda_a}$ peut permettre d'obtenir une image d'absorbance $Abs_{\lambda_a}$ de l'échantillon, à la longueur d'onde d'absorption $\lambda_a$, telle que:

$$Abs_{\lambda_a}(x,y) = -ln\left(\frac{I_{\lambda_a}(x,y)}{I_{0,\lambda_a}(x,y)}\right) (2)$$

où

- $Abs_{\lambda_a}(x,y)$ est la valeur de l'image d'absorbance $Ab_{\lambda_a}$ aux coordonnées $(x,y)$ ;
- $I_{\lambda_a}(x,y)$ et $I_{0,\lambda_a}(x,y)$ sont respectivement les intensités des pixels de l'image d'absorption et de fond, aux coordonnée $(x,y)$. Ces images peuvent être corrigées de l'image de noir du capteur d'image.

Les coordonnées $(x,y)$ sont définies dans le plan de détection $P_{20}$. Ce dernier étant parallèle au plan de l'échantillon $P_{10}$, les coordonnées $(x,y)$ correspondent également à des coordonnées dans le plan de l'échantillon $P_{10}$.

A partir de l'image d'absorbance $Abs_{\lambda_a}$, il est possible d'estimer une quantité $Q(x,y)$ d'analyte à chaque coordonnée $(x, y)$, avec

$$Q(x,y) = -\frac{Abs_{\lambda_a}(x,y)}{\mu_{\lambda_a}\varepsilon(x,y)} \quad (3),$$

où :

- $\varepsilon(x,y)$ est l'épaisseur de l'échantillon, selon l'axe de propagation Z, au niveau des coordonnées $(x, y)$ ;
- $\mu_{\lambda_a}$ est le coefficient d'absorption de l'analyte, par unité de longueur, à la longueur d'onde $\lambda_a$.

Ces étapes sont résumées sur la figure 3B :

Etape 100 : acquisition d'une image d'absorption $I_{\lambda_a}$, lorsque l'échantillon est illuminé par une source de lumière à la longueur d'onde d'absorption $\lambda_a$.

Etape 110 : acquisition d'une image de fond $I_{0,\lambda_a}$, sans échantillon entre le capteur d'image et la source de lumière, à la longueur d'onde d'absorption $\lambda_a$.

Etape 120 : calcul d'un ratio entre l'image d'absorption et l'image de fond, pour obtenir une image d'absorbance de l'échantillon $Abs_{\lambda_a}$, à la longueur d'onde d'absorption $\lambda_a$.

Selon une variante, l'échantillon est illuminé selon une longueur d'onde de base $\lambda_b$, à laquelle l'absorption du faisceau incident par l'échantillon est inférieure à l'absorption à la longueur d'onde d'absorption $\lambda_a$. Le procédé comporte alors les étapes suivantes :

Etape 130 : acquisition d'une image d'absorption $I_{\lambda_b}$, lorsque l'échantillon est illuminé par une source de lumière à la longueur d'onde de base $\lambda_b$.

Etape 140 : acquisition d'une image de fond $I_{0,\lambda_b}$, sans échantillon entre le capteur d'image et la source de lumière, à la longueur d'onde de base $\lambda_b$.

Etape 150 : calcul d'un ratio entre l'image d'absorption et l'image de fond, pour obtenir une image d'absorbance de l'échantillon $Abs_{\lambda_b}$, à la longueur d'onde de base $\lambda_b$.

Etape 160 : soustraction de l'image d'absorbance de l'échantillon à la longueur d'onde de base, à l'image d'absorbance de l'échantillon à la longueur d'onde d'absorption, de façon à obtenir une image $I$, représentative de l'absorbance due à l'analyte. $I = Abs_{\lambda_a} - Abs_{\lambda_b}$.

Cependant, il peut être difficile d'estimer des quantités d'analyte de façon quantitative. Les inventeurs estiment qu'il peut être préférable d'effectuer des comparaisons entre des images d'absorbance résultant de différents analytes, et par exemple de différents biomarqueurs.

On sait que l'activité cancéreuse peut être caractérisée par un indicateur morphologique, représentatif d'un ratio entre le volume du noyau et le volume du cytoplasme dans l'échantillon. En effet, il est connu que les cellules cancéreuses ont une activité métabolique plus importante que les cellules saines. De ce fait, elles tendent à avoir un noyau dont le volume est plus important que celui des cellules saines. De ce fait, le ratio volume nucléaire sur volume du cytoplasme est un indicateur utilisé par les histo-pathologistes pour établir le caractère malin d'une tumeur.

La publication Amrania H "Digistain : a digital staining instrument for histopathology", Optics Express 7299, Vol. 20, No. 7, 26 March 2012, décrit une méthode basée sur une comparaison de l'absorbance due à des groupes $PO_2^-$, représentatif de liaisons phosphodiester à l'intérieur du noyau cellulaire, et de l'absorbance due à des liaisons Amide, ces dernières étant représentatives de liaisons peptidiques, à l'intérieur du cytoplasme. En effectuant une comparaison entre l'absorbance due aux liaisons phosphodiester et l'absorbance due aux liaisons peptidiques, on peut obtenir un indicateur morphologique représentant un ratio volume nucléaire / volume cytoplasmique.

Les inventeurs se sont inspirés de cette méthode. Pour cela, ils ont illuminé successivement un échantillon, tel décrit en lien avec la figure 2A, selon différentes longueurs d'onde $\lambda_{a,1}$, $\lambda_{a,2}$, $\lambda_{b,1}$ et $\lambda_{b,2}$, telles que :

- $1/\lambda_{a,1}$ = 1081 cm$^{-1}$, ce qui correspond à un pic d'absorption de PO$_2^-$;
- $1/\lambda_{a,2}$ = 1654 cm$^{-1}$, ce qui correspond à un pic d'absorption de d'un groupement Amide, dans une bande spectrale communément désignée Amide I, correspondant à une vibration de la liaison C=O.
- $1/\lambda_{b,1}$ = 951 cm$^{-1}$, ce qui correspond à la ligne de base autour du pic d'absorption de PO$_2^-$;
- $1/\lambda_{b,2}$ = 1491 cm$^{-1}$, ce qui correspond à la ligne de base autour du pic d'absorption Amide I.

La source de lumière utilisée comportait 4 lasers QCL, émettant respectivement dans les plages spectrales suivantes :

- 1949 cm$^{-1}$ à 1706 cm$^{-1}$ ;
- 1712 cm$^{-1}$ à 1410 cm$^{-1}$ ;
- 1464 cm$^{-1}$ à 1149 cm$^{-1}$ ;
- 1218 cm$^{-1}$ à 896 cm$^{-1}$.

A chaque longueur d'onde $\lambda$, deux images ont été acquises :

- une image de fond $I_{0,\lambda}$, sans échantillon entre la source de lumière et le capteur d'image;
- une image d'absorption $I_\lambda$, l'échantillon étant disposé entre la source de lumière et le capteur d'image.

En effectuant un ratio entre l'image d'absorption $I_\lambda$ et l'image de fond $I_{0,\lambda}$, on a obtenu, à chaque longueur d'onde $\lambda$, des images d'absorbance $Abs_\lambda$.

On a ainsi obtenu, pour chaque analyte, en l'occurrence pour PO$_2^-$ et la liaison Amide :

- une image d'absorbance au niveau de chaque pic d'absorption; ces images étant notées $Abs_{\lambda_{a,1}}$ et $Abs_{\lambda_{a,2}}$;
- une image d'absorbance au niveau de la ligne de base s'étendant de part et d'autre de chaque pic d'absorption, ces images étant notées $Abs_{\lambda_{b,1}}$ et $Abs_{\lambda_{b,2}}$;

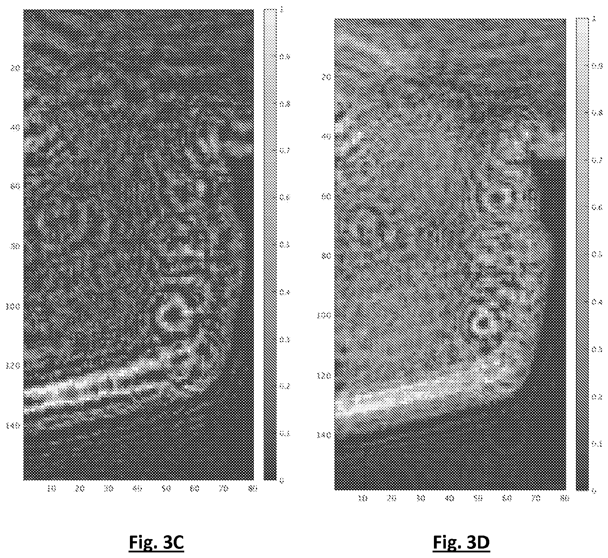

Les figures 3C et 3D représentent une image d'absorbance aux longueurs d'onde 1081 cm$^{-1}$ et 1654 cm$^{-1}$.

On a ensuite soustrait, à l'image d'absorbance obtenue à chaque longueur d'onde d'absorption, l'image d'absorbance obtenue à une longueur d'onde de base, de façon à obtenir une image, représentant une absorbance due de chaque analyte. Dans cet exemple, les analytes considérés sont PO$_2^-$ et un groupement amide.

Les images des absorbances dues à chaque analyte $I_1$ et $I_2$ correspondant respectivement à PO$_2^-$ et à la liaison amide, sont telles que :

$$I_1 = Abs_{\lambda_{a,1}} - Abs_{\lambda_{b,1}} \quad (4)$$

$$I_2 = Abs_{\lambda_{a,2}} - Abs_{\lambda_{b,2}} \quad (4')$$

Ce mode de réalisation revient à effectuer les étapes 100 à 160 décrites en lien avec la figure 3B, une première fois en considérant les longueurs d'onde $\lambda_{a,1}$ et $\lambda_{b,1}$, et une deuxième fois en considérant les longueurs d'onde $\lambda_{a,2}$ et $\lambda_{b,2}$.

On peut alors effectuer un ratio $\frac{I_1}{I_2}$ entre deux images $I_1$ et $I_2$, de façon à obtenir une cartographie du ratio volume nucléaire / volume cytoplasmique. En fonction du ratio $\frac{I_1(x,y)}{I_2(x,y)}$, on définit, sur l'échantillon, des régions d'intérêt correspondant à des zones tumorales.

Comme le montrent les exemples qui précèdent, l'invention permet de définir, sans marquage, des régions d'intérêt d'un échantillon, susceptibles de présenter un caractère pathologique. La configuration d'imagerie sans lentille permet d'adresser un champ d'observation élevé.

**Revendications**

1. Procédé d'observation d'un échantillon biologique (10), l'échantillon étant disposé entre une source de lumière (11) et un capteur d'image pixellisé (20), la source de lumière émettant un faisceau lumineux incident (12), se propageantt jusqu'à l'échantillon selon un axe de propagation (Z), et selon une longueur d'onde d'émission ($\lambda$) comprise entre 1 $\mu$m et 20 $\mu$m, le procédé comportant les étapes suivantes :

   a) illumination de l'échantillon par la source de lumière ;
   b) acquisition d'une image ($I_\lambda$) de l'échantillon par le capteur d'image pixelisé (20), aucune optique de formation d'image n'étant disposée entre l'échantillon et le capteur d'image;

   le procédé étant tel que :

   - l'échantillon (10) est apte à absorber une partie du faisceau lumineux incident, de telle sorte que l'image acquise ($I_\lambda$) est représentative d'une absorption du faisceau incident (12) par l'échantillon, à la longueur d'onde d'émission;

- la source de lumière éclaire une surface de l'échantillon supérieure à 1 mm$^2$ ou supérieure à 5 mm$^2$ ;
- l'image de l'échantillon acquise par le capteur d'image (20) correspond à une surface d'échantillon supérieure à 1 mm$^2$ ou supérieure à 5 mm$^2$ ;
- les pixels du capteur d'image définissent un plan de détection ($P_{20}$), l'échantillon étant disposé à une distance du plan de détection inférieure à 1 mm.

2. Procédé selon la revendication 1, comportant également les étapes suivantes :

   c) illumination du capteur d'image (20) par la source de lumière (11), à la longueur d'onde d'émission, sans échantillon entre le capteur d'image et la source de lumière, de façon à obtenir une image de fond ($I_{0,\lambda}$) ;
   d) comparaison de l'image ($I_\lambda$) acquise lors de l'étape b) et de l'image de fond ($I_{0,\lambda}$) acquise lors de l'étape c) pour obtenir une image ($Abs_\lambda$) de l'absorbance de l'échantillon à la longueur d'onde d'émission.

3. Procédé selon la revendication 2, dans lequel la longueur d'onde d'émission est une longueur d'onde d'absorption ($\lambda_a$) d'un analyte, correspondant à un pic d'absorption de l'analyte, le procédé comportant une cartographie d'une quantité de l'analyte dans l'échantillon à partir de l'image de l'absorbance ($Abs_\lambda$) à la longueur d'onde d'émission.

4. Procédé selon la revendication 2, dans lequel la longueur d'onde d'émission est une longueur d'onde d'absorption d'un analyte ($\lambda_a$), correspondant à un pic d'absorption de l'analyte, de façon à obtenir une image de l'absorbance de l'échantillon à la longueur d'onde d'absorption ($Abs_{\lambda_a}$), le procédé comportant également les étapes suivantes :

   e) illumination de l'échantillon selon une longueur d'onde de base ($\lambda_b$), à laquelle l'absorption de l'analyte est inférieure à l'absorption de l'analyte à la longueur d'onde d'absorption ($\lambda_a$) ;
   f) acquisition d'une image ($I_{\lambda_b}$) de l'échantillon par le capteur d'image pixelisé (20) ;
   g) illumination du capteur d'image (20) par la source de lumière (11), à la longueur d'onde de base, sans échantillon entre le capteur d'image et la source de lumière, de façon à obtenir une image de fond ($I_{0,\lambda_b}$) à la longueur d'onde de base ;
   h) comparaison de l'image ($I_{\lambda_b}$) acquise lors de l'étape f) et de l'image de fond ($I_{0,\lambda_b}$) acquise lors de l'étape g) pour obtenir une image ($Abs_{\lambda_b}$) de l'absorbance de l'échantillon à la longueur d'onde de base ($\lambda_b$) ;

le procédé comportant également une soustraction des images d'absorbance de l'échantillon respectivement à la longueur d'onde d'absorption et à la longueur d'onde de base ($Abs_{\lambda_a}, Abs_{\lambda_b}$), de manière à obtenir une image d'absorbance ($I$) due à l'analyte.

5. Procédé selon la revendication 4, dans lequel :

   les étapes a) à d) sont répétées en illuminant successivement l'échantillon selon :

   - une première longueur d'onde d'absorption ($\lambda_{a,1}$), correspondant à une longueur d'onde d'absorption d'un premier analyte ;
   - une deuxième longueur d'onde d'absorption ($\lambda_{a,2}$), correspondant à une longueur d'onde d'absorption d'un deuxième analyte ;

   de manière à obtenir des images de l'absorbance de l'échantillon ($Abs_{\lambda_{a,1}}, Abs_{\lambda_{a,2}}$) respectivement à la première longueur d'onde d'absorption et à la deuxième longueur d'onde d'absorption ;
   et dans lequel les étapes e) à h) sont répétées en illuminant successivement l'échantillon selon:

   - une première longueur d'onde de base ($\lambda_{b,1}$), à laquelle l'absorption du premier analyte est inférieure à l'absorption du premier analyte à la première longueur d'onde d'absorption ($\lambda_{a,1}$);
   - une deuxième longueur d'onde de base ($\lambda_{b,2}$), à laquelle l'absorption du deuxième l'analyte est inférieure à l'absorption du deuxième analyte à la deuxième longueur d'onde d'absorption ($\lambda_{a,2}$);

   de manière à obtenir des images de l'absorbance de l'échantillon ($Abs_{\lambda_{b,1}}, Abs_{\lambda_{b,2}}$) respectivement à la première longueur d'onde de base et à la deuxième longueur d'onde de base ;
   le procédé comportant également

   - une soustraction des images d'absorbance de l'échantillon respectivement à la première longueur d'onde d'absorption et à la première longueur d'onde de base ($Abs_{\lambda_{a,1}}, Abs_{\lambda_{b,1}}$), de manière à obtenir une image d'absorbance due au premier analyte ($I_1$);
   - une soustraction des images d'absorbance de l'échantillon respectivement à la deuxième longueur d'onde d'absorption et à la deuxième longueur d'onde de base ($Abs_{\lambda_{a,2}}, Abs_{\lambda_{b,2}}$), de manière à obtenir une image d'absorbance due au deuxième analyte ($I_2$);
- une comparaison de l'image d'absorbance due au premier analyte et de l'image d'absorbance due au deuxième analyte.

**6.** Procédé selon l'une quelconque des revendications précédentes, dans lequel l'échantillon est une lame de tissu biologique.

**7.** Procédé selon l'une quelconque des revendications précédentes, comportant, à partir de l'image acquise, une étape de détermination d'au moins une région d'intérêt de l'échantillon.

**8.** Procédé selon l'une quelconque des revendications précédentes, dans lequel l'échantillon (10) est maintenu par un support (15) comportant au moins un des matériaux suivants :

- silicium ;
- et/ou germanium ;
- et/ou fluorure de calcium ;
- et/ou fluorure de baryum.

**9.** Procédé selon l'une quelconque des revendications précédentes, dans lequel le capteur d'image pixelisé est formé d'une matrice de bolomètres.

**Patentansprüche**

**1.** Verfahren zur Beobachtung einer biologischen Probe (10), wobei die Probe zwischen einer Lichtquelle (11) und einem pixelierten Bildsensor (20) angeordnet ist, wobei die Lichtquelle einen einfallenden Lichtstrahl (12) aussendet, der sich bis zur Probe entlang einer Ausbreitungsachse (Z) und gemäß einer Emissionswellenlänge ($\lambda$) zwischen 1 $\mu$m und 20 $\mu$m ausbreitet, wobei das Verfahren die folgenden Schritte umfasst:

a) Beleuchtung der Probe durch die Lichtquelle ;
b) Erfassen eines Bildes ($I_\lambda$) der Probe durch den pixelierten bildsensor (20), wobei keine abbildende Optik zwischen der Probe und dem Bildsensor angeordnet ist;

wobei das Verfahren so ist, dass :

- die Probe (10) geeignet ist, einen Teil des einfallenden Lichtstrahls zu absorbieren, so dass das erfasste Bild ($I_\lambda$) repräsentativ für eine Extinktion des einfallenden Strahls (12) durch die Probe bei der Emissionswellenlänge ist;
- die Lichtquelle eine Fläche der Probe beleuchtet, die größer als 1 mm$^2$ oder größer als 5 mm$^2$ ist;

- das von dem Bildsensor (20) erfasste Bild der Probe einer Probenfläche von mehr als 1 mm$^2$ oder mehr als 5 mm$^2$ entspricht;
- die Pixel des Bildsensors eine Erkennungsebene ($P_{20}$) definieren, wobei die Probe in einem Abstand von weniger als 1 mm von der Erkennungsebene angeordnet ist.

**2.** Verfahren nach Anspruch 1, das auch die folgenden Schritte umfasst:

c) Beleuchten des Bildsensors (20) durch die Lichtquelle (11) bei der Emissionswellenlänge, ohne Muster zwischen dem Bildsensor und der Lichtquelle, um ein Hintergrundbild zu erhalten ($I_{0,\lambda}$) ;
d) Vergleich des in Schritt b) gewonnenen Bildes ($I_\lambda$) und des in Schritt c) gewonnenen Hintergrundbildes ($I_{0,\lambda}$), um ein Bild ($Abs_\lambda$) des Extinktion grades der Probe bei der Emissionswellenlänge zu erhalten.

**3.** Verfahren nach Anspruch 2, wobei die Emissionswellenlänge eine Absorptionswellenlänge ($\lambda_a$) eines Analyten ist, die einem Absorptionspeak des Analyten entspricht, wobei das Verfahren ein Abbilden einer Menge des Analyten in der Probe anhand des Bildes der Extinktion ($Abs_\lambda$) bei der Emissionswellenlänge umfasst.

**4.** Verfahren nach Anspruch 2, wobei die Emissionswellenlänge eine Absorptionswellenlänge eines Analyten ($\lambda_a$) ist, die einem Absorptionspeak des Analyten entspricht, so dass ein Bild der Extinktion der Probe bei der Absorptionswellenlänge ($Abs_{\lambda_a}$) erhalten wird, wobei das Verfahren auch die folgenden Schritte umfasst:

e) Beleuchten der Probe mit einer Basiswellenlänge ($\lambda_b$), bei der die Extinktion des Analyten geringer ist als die Extinktion des Analyten bei der Absorptionswellenlänge ($\lambda_a$);
f) Erfassen eines Bildes ($I_{\lambda_b}$) der Probe durch den pixelierten Bildsensor (20) ;
g) Beleuchten des Bildsensors (20) durch die Lichtquelle (11) bei der Basiswellenlänge, ohne Muster zwischen dem Bildsensor und der Lichtquelle, um ein Hintergrundbild ($I_{0,\lambda_b}$) bei der Basiswellenlänge zu erhalten;
h) Vergleich des in Schritt f) erfassten Bildes ($I_{\lambda_b}$) und des in Schritt g) erfassten Hintergrundbildes ($I_{0,\lambda_b}$), um ein Bild ($Abs_{\lambda_b}$) der Extinktion der Probe bei der Basiswellenlänge ($\lambda_b$) zu erhalten;

wobei das Verfahren auch eine Subtraktion der Extinktionsbilder der Probe bei der Absorptionswellenlänge bzw. der Basiswellenlänge ($Abs_{\lambda_a}$,$Abs_{\lambda_b}$) umfasst, um ein Extinktionsbild ($I$) aufgrund des Analyten zu erhalten.

**5.** Verfahren nach Anspruch 4, wobei :

die Schritte a) bis d) werden wiederholt, indem die Probe nacheinander beleuchtet wird gemäß:

- eine erste Absorptionswellenlänge ($\lambda_{a,1}$), die einer Absorptionswellenlänge eines ersten Analyten entspricht;
- eine zweite Absorptionswellenlänge ($\lambda_{a,2}$), die einer Absorptionswellenlänge eines zweiten Analyten entspricht;

so dass Bilder der Extinktion der Probe ($Abs_{\lambda_{a,1}}$, $Abs_{\lambda_{a,2}}$) jeweils bei der ersten Absorptionswellenlänge und der zweiten Absorptionswellenlänge erhalten werden;
und wobei die Schritte e) bis h) wiederholt werden, indem die Probe nacheinander gemäß beleuchtet wird:

- eine erste Basiswellenlänge ($\lambda_{b,1}$), bei der die Extinktion des ersten Analyten geringer ist als die Extinktion des ersten Analyten bei der ersten Absorptionswellenlänge ($\lambda_{a,1}$);
- eine zweite Basiswellenlänge ($\lambda_{b,2}$), bei der die Extinktion des zweiten Analyten geringer ist als die Extinktion des zweiten Analyten bei der zweiten Absorptionswellenlänge ($\lambda_{a,2}$);

um Bilder der Extinktion der Probe ($Abs_{\lambda_{b,1}}$, $Abs_{\lambda_{b,2}}$) jeweils bei der ersten Basiswellenlänge und der zweiten Basiswellenlänge zu erhalten; das Verfahren umfasst außerdem

- eine Subtraktion der Extinktionsbilder der Probe bei der ersten Absorptionswellenlänge bzw. der ersten Basiswellenlänge ($Abs_{\lambda_{a,1}}$, $Abs_{\lambda_{b,1}}$), um ein Extinktionsbild zu erhalten, das auf den ersten Analyten zurückzuführen ist ($I_1$) ;
- eine Subtraktion der Extinktionsbilder der Probe bei der zweiten Absorptionswellenlänge bzw. der zweiten Basiswellenlänge ($Abs_{\lambda_{a,2}}$, $Abs_{\lambda_{b,2}}$), um ein Extinktionsbild aufgrund des zweiten Analyten zu erhalten ;($I_2$)
- einen Vergleich des Extinktionsbildes aufgrund des ersten Analyten und des Extinktionsbildes aufgrund des zweiten Analyten.

**6.** Verfahren nach einem der vorhergehenden Ansprüche, wobei es sich bei der Probe um einen Objektträger aus biologischem Gewebe handelt.

**7.** Verfahren nach einem der vorhergehenden Ansprüche, das aus dem erfassten Bild einen Schritt zur Bestimmung mindestens eines interessierenden Bereichs der Probe umfasst.

**8.** Verfahren nach einem der vorhergehenden Ansprüche, bei dem die Probe (10) von einem Träger (15) gehalten wird, der mindestens eines der folgenden Materialien umfasst:

- Silizium ;
- und/oder Germanium ;
- und/oder Kalziumfluorid ;
- und/oder Bariumfluorid.

**9.** Verfahren nach einem der vorhergehenden Ansprüche, bei dem der pixelierte Bildsensor aus einer Bolometermatrix gebildet wird.

**Claims**

**1.** A method for observing a biological sample (10), the sample being placed between a light source (11) and a pixelated image sensor (20), the light source emitting an incident light beam (12), which propagates to the sample along a propagation axis (Z), and at an emission wavelength ($\lambda$) comprised between 1 $\mu$m and 20 $\mu$m, the method comprising the following steps:

a) illuminating the sample with the light source;
b) acquiring an image ($I_\lambda$) of the sample with the pixelated image sensor (20), no image-forming optic being placed between the sample and the image sensor;

the method being such that:

- the sample (10) is able to absorb some of the incident light beam, such that the acquired image ($I_\lambda$) is representative of an absorption of the incident beam (12) by the sample, at the emission wavelength;
- the light source illuminates an area of the sample larger than 1 mm$^2$ or larger than 5 mm$^2$;
- the image acquired of the sample by the image sensor (20) corresponds to an area of sample larger than 1 mm$^2$ or larger than 5 mm$^2$;
- the pixels of the image sensor define a detection plane ($P_{20}$), the sample being placed at a distance from the detection plane smaller than 1 mm.

**2.** The method as claimed in claim 1, also comprising the following steps:

c) illuminating the image sensor (20) with the light source (11), at the emission wavelength, with no sample between the image sensor and the light source, so as to obtain a background image ($I_{0,\lambda}$);

d) comparing the image ($I_\lambda$) acquired in step b) and the background image ($I_{0,\lambda}$) acquired in step c) to obtain an image ($Abs_\lambda$) of the absorbance of the sample at the emission wavelength.

3. The method as claimed in claim 2, wherein the emission wavelength is an absorption wavelength ($\lambda_a$) of an analyte, corresponding to an absorption peak of the analyte, the method comprising mapping an amount of the analyte in the sample on the basis of the image of the absorbance ($Abs_\lambda$) at the emission wavelength.

4. The method as claimed in claim 2, wherein the emission wavelength is an absorption wavelength of an analyte ($\lambda_a$), corresponding to an absorption peak of the analyte, so as to obtain an image of the absorbance of the sample at the absorption wavelength ($Abs_{\lambda_a}$), the method also comprising the following steps:

e) illuminating the sample at a base wavelength ($\lambda_b$), at which the absorption of the analyte is lower than the absorption of the analyte at the absorption wavelength ($\lambda_a$);

f) acquiring an image ($I_{\lambda_b}$) of the sample with the pixelated image sensor (20);

g) illuminating the image sensor (20) with the light source (11), at the base wavelength, with no sample between the image sensor and the light source, so as to obtain a background image ($I_{0,\lambda_b}$) at the base wavelength;

h) comparing the image ($I_{\lambda_b}$) acquired in step f) and the background image ($I_{0,\lambda_b}$) acquired in step g) to obtain an image ($Abs_{\lambda_b}$) of the absorbance of the sample at the base wavelength ($\lambda_b$);

the method also comprising subtracting the absorbance images of the sample at the absorption wavelength and at the base wavelength ($Abs_{\lambda_a}, Abs_{\lambda_b}$), respectively, so as to obtain an image ($I$) of absorbance due to the analyte.

5. The method as claimed in claim 4, wherein:

steps a) to d) are repeated so as to successively illuminate the sample at:

- a first absorption wavelength ($\lambda_{a,1}$), corresponding to an absorption wavelength of a first analyte;
- a second absorption wavelength ($\lambda_{a,2}$), corresponding to an absorption wavelength of a second analyte;

so as to obtain images of the absorbance of the sample ($Abs_{\lambda_{a,1}}, Abs_{\lambda_{a,2}}$) at the first absorption wavelength and at the second absorption wavelength, respectively;

and wherein steps e) to h) are repeated so as to successively illuminate the sample at:

- a first base wavelength ($\lambda_{b,1}$), at which the absorption of the first analyte is lower than the absorption of the first analyte at the first absorption wavelength ($\lambda_{a,1}$);
- a second base wavelength ($\lambda_{b,2}$), at which the absorption of the second analyte is lower than the absorption of the second analyte at the second absorption wavelength ($\lambda_{a,2}$);

so as to obtain images of the absorbance of the sample ($Abs_{\lambda_{b,1}}, Abs_{\lambda_{b,2}}$) at the first base wavelength and at the second base wavelength, respectively;

the method also comprising

- subtracting the images of absorbance of the sample at the first absorption wavelength and at the first base wavelength ($Abs_{\lambda_{b,1}}, Abs_{\lambda_{b,1}}$), respectively, so as to obtain an image ($I_1$) of absorbance due to the first analyte;
- subtracting the images of absorbance of the sample at the second absorption wavelength and at the second base wavelength ($Abs_{\lambda_{a,2}}, Abs_{\lambda_{b,2}}$), respectively, so as to obtain an image ($I_2$) of absorbance due to the second analyte;
- comparing the image of absorbance due to the first analyte and the image of absorbance due to the second analyte.

6. The method as claimed in any one of the preceding claims, wherein the sample is a slide of biological tissue.

7. The method as claimed in any one of the preceding claims, comprising, on the basis of the acquired image, a step of determining at least one region of interest of the sample.

8. The method as claimed in any one of the preceding claims, wherein the sample (10) is held by a carrier (15) comprising at least one of the following materials:

- silicon;
- and/or germanium;
- and/or calcium fluoride;
- and/or barium fluoride.

9. The method as claimed in any one of the preceding claims, wherein the pixelated image sensor is formed from a matrix-array of bolometers.

**Fig. 1**

**Fig. 2A**

Fig. 2B

**Fig. 3A**

**Fig. 3B**

**Fig. 3C**

**Fig. 3D**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20120122084 A1
- WO 2008090330 A
- WO 2016189257 A
- EP 3199941 A
- EP 3147646 A
- US 20050190286 A
- FR 3034197 A1

**Littérature non-brevet citée dans la description**

- A microfluidic shadow imaging system for the study of the nematode Caenorhabditis elegans in space. **LANGE D et al.** SENSORS AND ACTUATORS B: CHEMICAL. ELSEVIER BV, 29 June 2005, vol. 107, 904-914
- BOLOMETERS FOR INFRARED AND MILLIMETER WAVES. **RICHARDS P L**. JOURNAL OF APPLIED PHYSICS. AMERICAN INSTITUTE OF PHYSICS, 01 July 1994, vol. 76, 1-24
- **OZCAN AYDOGAN et al.** Lensless Imaging and Sensing. *Annual Review of Biomedical Engineering*, 11 July 2016, vol. 18 (1), ISSN 1523-9829, 77-102, https://www.annualreviews.org/doi/pdf/10.1146/annurev-bioeng-092515-010849
- **AMRANIA H**. Digistain : a digital staining instrument for histopathology. *Optics Express 7299*, 26 March 2012, vol. 20 (7)